Europäisches Patentamt

(19) European Patent Office

Office européen des brevets

(11) Publication number: **0 007 744**
**B1**

# EUROPEAN PATENT SPECIFICATION

(45) Date of publication of patent specification: **03.02.82**

(51) Int. Cl.³: **G 01 N 33/58,**
**G 01 N 33/78**

(21) Application number: **79301365.7**

(22) Date of filing: **11.07.79**

(54) **Assay for thyroid hormone binding capacity.**

(30) Priority: **12.07.78 US 923712**

(43) Date of publication of application:
**06.02.80 Bulletin 80/3**

(45) Publication of the grant of the European patent:
**03.02.82 Bulletin 82/5**

(84) Designated Contracting States:
**BE DE GB IT NL SE**

(56) References cited:
**US - A - 3 888 629**
**US - A - 3 896 217**
**US - A - 4 005 187**
**US - A - 4 009 005**
**US - A - 4 032 626**
**US - A - 4 059 685**
**US - A - 4 108 976**

(73) Proprietor: **Becton, Dickinson and Company**
**Mack Centre Drive**
**Paramus, New Jersey 07652 (US)**

(72) Inventor: **Reese, Max G.**
**977 E. 5600 South**
**Salt Lake City, Utah (US)**
Inventor: **Johnson, LaVell R.**
**3201, Teton Drive**
**Salt Lake City, Utah (US)**

(74) Representative: **Ruffles, Graham Keith**
**MARKS & CLERK 57-60 Lincoln's Inn Fields**
**London WC2A 3LS (GB)**

Courier Press, Leamington Spa, England.

# 0 007 744

Assay for thyroid hormone binding capacity

The present invention relates to an assay, and more particularly to an assay for qualitatively determining the thyroid hormone binding capacity of a serum sample.

Human serum contains proteins which are known to bind thyroid hormones; in particular, thyroxine binding globulin (TBG) thyroxine binding prealbumin (TBPA) and albumin. The thyroid hormones (thyroxine ($T_4$) and triiodothyronine ($T_3$)) are primarily bound to TBG.

A qualitative determination of changes in the concentration of the thyroid hormone binding capacity, in particular, TBG is useful in evaluating various factors such as hypothyroidism, the effect on TBG resulting from pregnancy estrogen administration, etc. As specific instances, the total TBG is raised by estrogen administration and lowered by nephritis.

In US Patent No. 4,005,187 there is described a diagnostic test method which involves allowing two solvents to form a solid absorbent in a test fluid mixture which contains a diagnostically significant component. The solid absorbent, usually an inorganic precipitate, absorbs at least a portion of the diagnostically significant component from the test fluid mixture, and can be separated for subsequent measurement of the amount of the significant component.

The US Patent describes the application of this method to T-3 tests, that is, to the measurement of the free or unsaturated binding capacity of thyroid hormone binding globulin. In common with other known T-3 tests, the use of the solid absorbent formed *in situ* does not readily allow for automation or repeated use.

In accordance with the present invention, there is provided an automated assay for qualitative determination of the thyroid hormone binding capacity of a serum, in which assay a $T_3$ tracer is mixed with an unknown serum sample and caused to flow through a chamber containing a $T_3$ binder supported on a solid support. The amount of $T_3$ tracer which passes through the chamber is determined, followed by flowing of an eluting solution through the chamber to release any $T_3$ tracer which is bound to the binder in the chamber. The amount of $T_3$ tracer released from the binder is determined, thereby to determine the percent of $T_3$ tracer which was bound to the binder in the chamber. The percent of $T_3$ tracer bound in the sample is compared with the percent of $T_3$ tracer bound in a reference sample to qualitatively determine the $T_3$ binding capacity of the serum sample.

The assay provides a relative measure of the free TBG. As mentioned, TBG is the principle carrier protein in plasma or serum for $T_4$ and $T_3$. Without being bound by any theory, it seems that in the present assay, equilibrium is established between TBG with $T_4$, $T_3$, and tracer $T_3$ ($TT_3$). $T_4$ has a greater affinity to TBG than $T_3$, and thus the $TT_3$ does not displace appreciably any $T_4$ bound to TBG. The degree of binding of $TT_3$ to TBG is related to the degree of unsaturated, i.e. free, TBG which is available and which in hyperthyroid patients is low and in hypothyroid patients is high. TBG carries around 99.97% of the total $T_4$ in serum, and therefore a measure of total $T_4$ does not reflect accurately the metabolically active free $T_4$ concentration in serum. When taken with the total $T_4$, a measurement of the relative binding capacity of the free TBG by using an assay according to the present invention also allows a corrected interpretation of the free $T_4$ in a qualitative sense.

The binder or receptor which is bound to the solid support for use in the present assay may be any one of a wide variety of thyroid hormone binders, including antibodies, serum proteins and the like. The preferred binder is an antibody.

The $T_3$ tracer may be any one of a wide variety of tracers in which $T_3$ or an appropriate analogue thereof includes a label or tag such as a radioactive isotope, enzyme, fluorescent tag, etc. The preferred tracer has a radioactive isotope as the lable or tag, in particular a radioiodine tag.

The binder may be covalently bound to a suitable support, as described in US Patent No. 4,059,685 or in US Patent No. 4,108,975, and the reader is referred to these two documents for details. As described in such documents, the particulate support contains a substrate which is superficially porous refractory particle made up of discrete macroparticles with impervious non-porous cores, having joined thereto a coating of a series of sequentially adsorbed, like monolayers of like inorganic microparticles. The core, in the form of a sphere is of a diameter of between 5 and 500 microns and is composed of glass, although it may be of sand, ceramic or the like. Affixed to the core is a plurality of layers of microparticles which form an outer porous coating. The microparticles may range in size from 5 millimicrons to 1 micron. The microparticles may be amorphous silica, alumina, thoria, or the like. A preferred substrate is that known as Zipax, the nature of which is mentioned in USA Patent No. 4,059,685 in column 4 ("Zipax" being a Trade Mark).

As described in the prior art, the substrate has adhered thereto a polymer preferably a water-insoluble polymer, with such polymer being activated or provided with functional groups for covalent binding of the receptor. Such supports, as hereinabove noted, are more fully described elsewhere, and accordingly no further details are necessary here for a complete understanding of the present invention.

Alternatively, the solid support can be fibrous cotton, and can be woven e.g. a woven cotton gauze, or non-woven e.g. balls of cotton, with the cotton preferably being a woven gauze. The cotton is most preferably an absorbent cotton and in particular mercerized absorbent cotton; such a cotton has

0 007 744

greater binder capacity. Thus if the cotton has not been mercerized it is preferably treated with sodium hydroxide or the like reagent before use.

A $T_3$ binder may be conveniently supported on a cotton substrate by effecting activation of the substrate with cyanogen bromide using any of the procedures known generally in the art, followed by conjugation, normally through covalent linking, of the binder to the cotton. In brief, a typical procedure involves contacting the cotton substrate with cyanogen bromide at a basic pH, generally in the order of from pH 10 to pH 12, followed by washing and air-drying. The binder or receptor to be used in the assay may then be covalently bonded to the thus activated cotton substrate by any of the procedures known in the art; see, for example, the procedures described in US Patents No. 4,059,685 and 4,108,976.

After being supported on a support, the binder is placed in a suitable flow-through chamber for effecting binding of free $T_3$ tracer included in the sample. The assay is effected automatically employing automated equipment providing for regeneration of the receptor, for example as described in detail in US Patent Nos. 3,896,217 and 4,009,005 to which the reader is referred.

More particularly, in accordance with a preferred process of the present invention, $T_3$ tracer, in particular radioiodinated $T_3(T_3{}^*)$, is mixed with a serum sample or a reference serum sample, and the resulting mixture passed through the chamber containing $T_3$ binder, in particular $T_3$ antibody covalently bound to the particulate support. $T_3$ tracer which is not bound to $T_3$ binding protein, in particular TBG present in the sample, is bound to the binder in the chamber. $T_3$ tracer bound to serum protein passes through the chamber. The radioactivity of the $T_3$ tracer which passes through the chamber is then counted.

Thereafter, the $T_3$ tracer bound to the immobilized receptor or binder in the chamber is released from the binder by flowing an eluting solution through the chamber to effect stoichiometric release of the $T_3$ tracer bound to the binder. The radioactivity of the released tracer is then counted.

Following elution, the binder is rinsed and then may be re-used for a further assay.

Thus, in a preferred assay using apparatus as described in USA Patent No. 4009005, the reference or sample is pre-incubated in the flow system with $^{125}I—T_3(T_3{}^*)$. Equilibrium is established between the $T_3{}^*$ and TBG in the serum (Equation 1). This equilibrated mixture flows over immobilized antibody ($Ab_{(ss)}$) and the unbound $T_3{}^*$ binds to the antibody. The $T_3{}^*$ which is bound to serum binding protein does not attach to the antibody but is counted by the gamma detector as the "free $T_3$" (Equation 2). An eluting agent then passes over the immobilized antibody releasing the bound $T_3{}^*$ which is counted as the bound fraction (Equation 3). The antibody is washed and ready to receive the next sample.

$$(1) \quad TBG_{(serum)}+T_3{}^* \quad \xrightleftharpoons{\text{auto dilution}} \quad TBG—T_3{}^*+T_3{}^*$$

$$(2) \quad TBG—T_3{}^*+T_3{}^*+Ab_{(ss)} \longrightarrow T_3{}^*—Ab_{(ss)}+TBG—T_3{}^*{}_{(Free)}$$

$$(3) \quad T_3{}^*—Ab_{(ss)} \longrightarrow Ab_{(ss)}+T_3{}^*{}_{(bound)}$$

The percent $T_3$ uptake, i.e. the percentage of $T_3$ tracer which was bound to the immobilized binder in the chamber, is then determined. This value is given by the ratio of $T_3$ eluted from the chamber to the sum of the $T_3$ passing through the chamber and the $T_3$ eluted subsequently from the chamber.

The percent binding of the $T_3$ tracer to the immobilized binder is inversely related to the serum protein binding capacity, and the binding capacity of a given serum sample is qualitatively determined by comparing the percentage of $T_3$ tracer bound in the unknown sample with the percentage of $T_3$ tracer bound in a reference sample.

For assays embodying the present invention, it is preferred that any inter-assay variations are minimized by utilizing a reference sample with an appropriately assigned percent uptake. The asigned percent uptake value of the reference serum is determined by reference to a normal pooled sample. In practice a normal serum pool has a percent uptake of about 30%.

Using a reference sample with assigned uptake, we have the equation (4):

$$(4) \quad \text{Percent Uptake(s)}=\frac{(\text{Percent Binding(s)}) \ (\text{Percent Uptake (R)})}{(\text{Percent Binding (R)})}$$

Where:

Percent Binding (s)=Percent binding of $TT_3$ to antibody in the unknown.

Percent Binding (R)=Percent binding of $TT_3$ to antibody in the reference serum.

Percent Uptake (R)=The assigned percent uptake for the reference serum.

The thus determined $T_3$ uptake is a qualitative measurement of the serum binding capacity of the unknown, with such binding capacity showing a qualitative increase when the $T_3$ uptake is below the assigned percent uptake, and a qualitative decrease when the $T_3$ uptake is above the assigned percent uptake.

The results can be expressed as a $T_3$ Ratio. This normalizes the percent uptake such that the mean is 1.0. Where the percent uptake for a normal pool is 30%, the $T_3$ ratio is given by equation (5):

$$(5) \; T_3 \; Ratio = \frac{Percent \; Uptake \; (s)}{30}$$

If total thyroxine values are also available the Free Thyroxine Index (FTI) can be obtained from the $T_3$ Ratio.

The invention is further described by way of the following example; however, the scope of the invention is not limited thereby:

Example

The reagents and equipment employed in the assay were in themselves conventional and were as follows:

(a) The adsorption buffer used to introduce the sample was $0.02M$ phosphate, pH 7.5 and $0.5M$ sodium chloride.

(b) The eluting solution was 50% methanol containing $0.1M$ glycinate, pH 10.5.

(c) The rinse solution was stabilized distilled water.

(d) The sample buffer was $0.2M$ phosphate, pH 7.5, $0.5M$ sodium chloride and 0.01% bovine serum albumin.

(e) The tracer was $^{125}I$-labeled $T_3$ antigen, specific activity 3,000 mC/mg.

(f) The antibody was prepared using $T_3$ specific antibody raised in rabbits against triiodothyropropionic acid conjugated to rabbit albumin.

(g) The $T_3$ Uptake Reference was stabilized human serum of known percent uptake value.

The assay was effected with the antibody covalently linked to either cyanogen bromide-activated, dextran-coated Zipax or cyanogen bromide-activated, mercerized absorbent cotton gauze. Automated equipment as described in US Patent No. 4009005 was employed. Such apparatus is commercially available from Becton, Dickinson Immunodiagnostics, Automated Immunochemistry Systems and is identified by the registered trade mark ARIA II.

The assay was effected employing both unknown sample and reference sample, with the reference uptake being assigned a value of 30%. A computer contained within the apparatus was used to process the results (though see the worked example below). All results were printed on a permanent record. The print-out identified each sample by number and showed the uptake in percent as normalized to the reference. It also gave the $T_3$ ratio.

A typical calculation performed by the computer was as follows:

Given that

Percent Uptake Reference=31.6
Percent Binding Reference=31.4

and having found that

Percent Uptake Sample=31.7
then from Equation (4)

$$T_3 \; Uptake = \frac{31.7 \times 36.6}{31.4}$$

$$=31.9$$

and from Equation (5)

$$T_3 \; Ratio = \frac{31.9}{30}$$

$$=1.06$$

Thus for the particular sample with a percent binding of 31.7 the computer printed out a $T_3$ Uptake of 37.9 and a $T_3$ Ratio of 1.06.

4

**0 007 744**

The present invention is particularly advantageous in that it is possible to determine automatically and qualitatively the thyroid hormone binding capacity of a serum sample. Moreover, such determination can be effected in a rapid manner.

Numerous modifications and variations of the present invention are possible.

## Claims

1. An automated assay for qualitative determination of the thyroid hormone binding capacity of a serum sample, comprising:

(a) mixing a known amount of $T_3$ tracer with serum sample;

(b) flowing the mixture from step (a) through a chamber containing a $T_3$ binder supported on a solid support;

(c) determining the amount of $T_3$ tracer which passes through the chamber;

(d) flowing an eluting solution through the chamber to release $T_3$ tracer bound to the binder in said chamber;

(e) determining the amount of $T_3$ tracer released from the binder;

(f) determining the percent of $T_3$ tracer which was bound to the binder; and

(g) comparing the percent of bound $T_3$ tracer in the sample with the percent of bound $T_3$ tracer in a reference sample to determine qualitatively the $T_3$ binding capacity of said serum sample.

2. An assay according to Claim 1 wherein the $T_3$ tracer is labelled with a radioisotope.

3. An assay according to Claim 2 wherein the radioisotope is $^{125}I$.

## Patentansprüche

1. Automatisierte Analyse zur qualitativen Bestimmung der Thyroidhormon-Bindungskapazität einer Serumprobe mit folgenden Schritten:

(a) man mischt eine bestimmte Menge einer markierten $T_3$-Substanz mit der Serumprobe;

b) man läßt die Mischung des Schrittes a) durch eine Kammer fließen, die einen auf einem festen Träger angeordneten $T_3$-Binder enthält;

c) man bestimmt die Menge der durch die Kammer fließenden markierten $T_3$-Substanz;

d) man läßt eine Spülflüssigkeit durch die Kammer fließen, um die an den Binder in der Kammer gebundene markierte $T_3$-Substanz abzulösen;

e) man bestimmt die vom Binder freigegebene Menge der markierten $T_3$-Substanz;

f) man bestimmt den Prozentsatz der markierten $T_3$-Substanz, der an den Binder gebunden war;

g) man vergleicht den Prozentsatz der in der Probe gebundenen, markierten $T_3$-Substanz mit dem Prozentsatz der markierten $T_3$-Substanz, die in einer Referenzprobe gebunden ist, um qualitativ die $T_3$-Bindungskapazität der Serumprobe zu bestimmen.

2. Analyse nach Anspruch 1, bei welcher die markierte $T_3$-Substanz mit einem Radioisotop markiert ist.

3. Analyse nach Anspruch 2, bei welcher das Radioisotop $^{125}I$ ist.

## Revendications

1. Tritrage automatisé pour la détermination qualitative du pouvoir de fixation des hormones thyroïdiennes d'un échantillon de sérum, caractérisé en ce que:

(a) on mélange une quantité connue de traceur de $T_3$ à l'échantillon de sérum,

(b) on fait s'écouler le mélange obtenu à l'étape (a) à travers une chambre contenant un fixateur de $T_3$ porté par un support solide,

(c) on détermine la quantité de traceur de $T_3$ qui passe à travers la chambre,

(d) on fait s'écouler une solution d'élution à travers la chambre pour libérer le traceur de $T_3$ lié au fixateur dans ladite chambre,

(e) on détermine la quantité de traceur de $T_3$ libérée à partir du fixateur,

(f) on détermine le pourcentage de traceur de $T_3$ qui était lié au fixateur et

(g) on compare le pourcentage de traceur de $T_3$ lié dans l'échantillon au pourcentage de traceur de $T_3$ lié dans un échantillon de référence afin de déterminer qualitativement le pouvoir de fixation de $T_3$ dudit échantillon de sérum.

2. Titrage suivant la revendication 1, caractérisé en ce que le traceur de $T_3$ est marqué à l'aide d'un radioisotope.

3. Titrage suivant la revendication 2, caractérisé en ce que le radioisotope est le $^{125}I$.

5